(19) Europäisches Patentamt
European Patent Office
Office européen des brevets

(11) **EP 2 432 387 B1**

(12) **EUROPÄISCHE PATENTSCHRIFT**

(45) Veröffentlichungstag und Bekanntmachung des Hinweises auf die Patenterteilung:
**23.11.2016 Patentblatt 2016/47**

(21) Anmeldenummer: **10721325.8**

(22) Anmeldetag: **17.05.2010**

(51) Int Cl.:
**A61B 5/06** (2006.01)     **A61B 34/20** (2016.01)

(86) Internationale Anmeldenummer:
**PCT/EP2010/002991**

(87) Internationale Veröffentlichungsnummer:
**WO 2010/133320 (25.11.2010 Gazette 2010/47)**

(54) **VERFAHREN ZUM ERZEUGEN VON POSITIONSDATEN EINES INSTRUMENTES**

METHOD FOR GENERATING POSITION DATA OF AN INSTRUMENT

PROCÉDÉ POUR GÉNÉRER DES DONNÉES DE POSITION D'UN INSTRUMENT

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **18.05.2009 DE 102009021705**

(43) Veröffentlichungstag der Anmeldung:
**28.03.2012 Patentblatt 2012/13**

(73) Patentinhaber: **Mucha, Dirk**
**10961 Berlin (DE)**

(72) Erfinder: **Mucha, Dirk**
**10961 Berlin (DE)**

(74) Vertreter: **Bobbert & Partner**
**Patentanwälte PartmbB**
**Postfach 1252**
**85422 Erding (DE)**

(56) Entgegenhaltungen:
**DE-A1-102004 017 834      US-A1- 2005 228 270**
**US-A1- 2006 122 497      US-A1- 2008 294 034**
**US-A1- 2010 121 174**

**Beschreibung**

Die vorliegende Erfindung betrifft ein Verfahren zum Erzeugen von Positionsdaten und zum Erkennen von Deformationen eines Instrumentes gemäß dem Oberbegriff des Anspruchs 1 sowie eine Vorrichtung gemäß dem Oberbegriff des Anspruchs 7, ein Computerprogramm-Produkt mit Programmcode gemäß Anspruch 13, ein Computerprogramm gemäß dem Anspruch 14 und ein digitales Speichermedium gemäß Anspruch 15.

Beim Arbeiten mit einem Instrument in einem Bereich, der nicht direkt einsehbar ist, kann es von Bedeutung sein, die aktuelle Lage des Instrumentes zu erfassen und in geeigneter Weise darzustellen. Bekannt ist, ein Instrument mit elektromagnetischen Lagesensoren, sogenannten Spulenelementen, zu versehen. Ein Feldgenerator, der in räumlicher Nähe des Instrumentes angeordnet ist, erzeugt ein elektromagnetisches Feld, das elektrische Spannungen in den Spulenelementen des Instrumentes nach dem Gesetz der elektromagnetischen Induktion (Induktionsgesetz) induziert. Die Höhe der induzierten Spannungen bzw. der elektrischen Ströme (Ohmsches Gesetz) in den Spulenelementen kann je nach räumlicher Lage und Positionierung der Sensoren an dem Instrument unterschiedlich sein.

Eine Steuerungseinheit, die mit dem Feldgenerator und den Lagesensoren verbunden ist, kann anschließend aus den verschiedenen Messdaten des Feldgenerators und der Lagesensoren die Position der Sensoren und damit des Instrumentes berechnen und beispielsweise auf einem Monitor darstellen.

Bekannt sind auch Verfahren zur Positionsbestimmung von Instrumenten, die auf optischen Messprinzipien von geeigneten Sensoren auf dem Instrument beruhen.

Einsetzbar sind derartige Verfahren für Instrumente, die starr sind und sich während der Anwendung der Instrumente nicht verformen. Werden derartige Verfahren bei Instrumenten eingesetzt, die sich während der Anwendung verformen, können die Positionsdaten nicht eindeutig von möglichen Störsignalen unterschieden werden.

Aus der DE 2004 017 834 A1 ist eine Kathetereinrichtung bekannt, umfassend einen Katheter zum Einführen in ein Hohlraumorgan, insbesondere ein Gefäß, mit mehreren entlang der Katheterlängsachse verteilt angeordneten separat ansteuerbaren Biegeelementen, sowie mehreren um die Katheterlängsachse verteilt angeordneten kommunizierenden Biegesensoren.

Aus der DE 10 2006 052 886 A1 ist ein System zum Erfassen der Form eines flexiblen Einführteils eines Endoskops bekannt, umfassend ein Positionserfassungssystem, das die Positionen beider Enden eines Biegeabschnittes des Einführteils erfasst, ein Mittel zum Bestimmen des Biegezustandes des Biegeabschnittes und einen Formwiedergabeprozessor zur Wiedergabe des Biegeabschnittes entsprechend den erfassten Positionen und dem Biegezustand.

Weitere Vorrichtungen und Verfahren zur Erzeugung von Positionsdaten von medizinischen Instrumenten sind z.B. aus US2008/0294034A1 und US2005/0228270A1 bekannt.

Aufgabe der vorliegenden Erfindung ist es, ein Verfahren vorzuschlagen, dass die Lagebestimmung mindestens eines Punktes von Instrumenten mit Lagesensoren erlaubt, die sich während der Anwendung verformen können.

Die vorliegende Erfindung wird gelöst durch die Merkmalskombination des Anspruchs 1.

Bei diesem Verfahren weist das Instrument wenigstens zwei verschiedene Abschnitte, einen ersten und einen zweiten Abschnitt auf. Die Form der beiden Abschnitte kann gleich oder unterschiedlich sein und wird je nach Anwendung verschieden ausgeführt. Jeder der wenigstens zwei Abschnitte weist zumindest jeweils einen Sensor auf.

Die Lage des zweiten Sensors, der sich auf oder in dem zweiten Abschnitt des Instrumentes befindet, wird in Bezug auf den ersten Abschnitt rechnerisch bestimmt, oder anders ausgedrückt mit einer mathematischen Gleichung oder eines Gleichungssystems, berechnet.

Vorteilhafte Weiterentwicklungen der erfindungsgemäßen Aufgabe sind jeweils Gegenstand der Unteransprüche.

Eine Verformung wird hier in manchen Ausführungsformen in dem Sinn beschrieben, dass der zweite Abschnitt seine Lage in Bezug auf den ersten Abschnitt verändert. Je nach Geometrie und Materialbeschaffenheit des Instrumentes, nach physikalischen Umgebungsparametern wie beispielsweise Temperatur und Druck sowie nach den voraussichtlich angreifenden Kräften und Momenten an dem Instrument kann es möglich sein, die voraussichtliche Verformung mathematisch zu beschreiben oder mit ausreichender Genauigkeit zu approximieren.

In bestimmten Ausführungsformen erfolgt das rechnerische Bestimmen der Lage des zweiten Sensors in Bezug auf den ersten Abschnitt auf mindestens zwei Arten, wobei eine Art nicht die messtechnisch bestimmte Lagen des ersten und zweiten Sensors verwendet, die während der Anwendung erfasst werden.

In manchen Ausführungsformen betrifft oder ist die erste Art die Herstellung des Bezugs mit mathematischen Methoden aus Lagendaten aus der messtechnische Erfassung des ersten und des zweiten Sensors zum Zeitpunkt der Anwendung.

In bestimmten Ausführungsformen betrifft oder ist die zweite Art eine mathematische Beschreibung der möglichen Lagen, die der zweite Sensor in Bezug auf den ersten Sensor einnehmen kann, wenn die mechanischen Einschränkungen, die die Verbindung zwischen ersten und zweiten Abschnitt liefert, beachtet werden. Die vollständige oder hinreichende mathematische Beschreibung ist zum Zeitpunkt der Anwendung vorhanden, z.B. in einer Speichereinrichtung hinterlegt.

Diese Beschreibung kann zum Beispiel eine formale mathematische Beschreibung in Form einer Gleichung einer Bahnkurve oder eine Tabelle mit diskreten Lagen. Lagen zwischen zwei Einträgen der Tabelle werden mittels Interpolationsverfahren ermittelt.

Die Beschreibung der Lage kann mittels

- messtechnischer Verfahren, und/oder
- Herleitung der Bahnkurve aus den mechanischen Eigenschaften des Instruments, und/oder
- Simulation mit mathematischen Verfahren

erfolgen.

"In Bezug auf den ersten Abschnitt" bedeutet, dass die Lage des zweiten Sensors relativ zum ersten Abschnitt, beispielsweise in Bezug auf einen bestimmten Punkt oder Bereich - wie etwa dem Ende des ersten Abschnitts - berechnet wird.

Durch Kenntnis der ermittelbaren Lagebeziehung zwischen dem ersten und dem zweiten Abschnitt des Instrumentes oder zwischen dem zweiten Sensor und dem ersten Abschnitt sind neben weiteren Vorteilen ein Prüfen auf Plausibilität erhaltener Lagedaten und/oder ein Berechnen der vorliegenden Deformation des Instrumentes und/oder ein Bestimmen der tatsächlichen Lage z. B. der Spitze oder des Werkzeugabschnitts des Instrumentes auch bei dessen einsatzbedingter Deformierung vorteilhaft möglich.

Die elektromagnetischen Lagesensoren, alle oder wenigstens einer, können in manchen Ausführungsformen so genannte Saturationskernmagnetometer sein, die ein Signal liefern, das proportional zur lokalen auftretenden Feldstärke des erzeugten elektromagnetischen Feldes ist.

Jeder der vorgesehenen Sensoren kann auf der Oberfläche des Instrumentes oder innerhalb des Instrumentes bzw. der Abschnitte angeordnet sein. Beispielsweise kann es vorteilhaft sein, wenigstens einen Sensor im Instrument zu kapseln, wenn er nicht mit der Umgebung in Berührung kommen soll. Dies kann entweder zum Schutz des Sensors vor dem Umgebungsmedium dienen, aber auch umgekehrt dem Schutz des Umgebungsmediums oder -gewebes vor dem Sensor. Mögliche Anwendungsfälle sind Messungen in toxischen und/ oder aggressiven Substanzen, die den Sensor zerstören könnten oder Anwendungen in der Umgebung von humanem oder tierischem Gewebe, das durch Sensormaterialien beeinflusst werden kann.

Die Sensoren, die auch als Lagesensoren bezeichnet werden können, können auf unterschiedlichen physikalischen Messprinzipien beruhend messen oder arbeiten.

Als bevorzugte Ausführungsform werden elektromagnetische Sensoren verwendet. Dies hat den Vorteil, dass beispielsweise gegenüber optischen Sensoren die Sensoren innerhalb verschiedener Medien, Gehäuse oder Verkapselungen verwendet werden können, ohne dass eine direkte Sichtverbindung notwendig ist. Im Umgang mit toxischen Substanzen oder in schwer zugänglichen Geräten und Maschinen ist eine Verkapselung oft notwendig.

Zu beachten ist andererseits die mögliche Störung des Mediums oder der Anwendungsumgebung durch die elektromagnetischen Felder. Daher können auch andere Messprinzipien für die Sensoren verwendet werden, beispielsweise optische Verfahren mittels Laserlicht.

Weitere Verfahren mit elektromagnetischen Wellenlängenbereichen, die beispielsweise nahe dem für den Menschen sichtbaren Spektrum (Licht) liegen, wie etwa Infrarot- oder Ultraviolett-Strahlung, können ebenfalls Anwendung finden.

Weiterhin können sich die Sensoren, die sich auf dem ersten Abschnitt befinden, von den Sensoren des zweiten Abschnitts unterscheiden. Dabei kann der Sensor auf dem ersten Abschnitt als Hauptsensor bezeichnet werden, der Sensor auf dem zweiten Abschnitt als Nebensensor, oder umgekehrt. Der Hauptsensor kann sich in den Abmaßen, in der Sensitivität, der Messgenauigkeit, den Gehäusematerialien des Sensors und anderen Merkmalen von dem Nebensensor unterscheiden. Auch die - insbesondere physikalischen - Messprinzipien von Haupt- und Nebensensor können unterschiedlich sein. Der Hauptsensor kann beispielsweise auf einem optischen Messprinzip beruhen, der Nebensensor beispielsweise auf einem elektromagnetischen Messprinzip.

Ebenfalls ist es möglich, dass ein Hauptsensor auf dem Instrument auf dem ersten Abschnitt angeordnet ist, und mehrere Nebensensoren auf dem zweiten Abschnitt. Im Falle mehrerer Nebensensoren kann für jeden ein eigenes Biegemodell oder eine eigene Biegelinie (entspricht der Kurve aller im Modell möglichen Lagen oder Positionen des jeweiligen Nebensensors) ermittelt und ggf. gespeichert sein. Die Biegemodelle und Biegelinien können sich voneinander unterscheiden.

Die messtechnische Bestimmung der Lage der Sensoren hängt von den verwendeten Messprinzipien der Sensoren ab und wird hier nicht weiter vertieft, da sie dem Fachmann hinreichend bekannt ist.

Variablen der mathematischen Gleichung(en) zur Lage-, bzw. Positionsbestimmung eines Punktes oder Bereichs des ersten Abschnitts in Bezug zu der Lage bzw. Position des zweiten Sensors sind vorzugsweise drei Variablen, die im Folgenden näher beschrieben werden. Die erste Variable bestimmt die Lage bzw. Position eines Punktes oder Bereichs des ersten Abschnitts in Bezug zu der Lage bzw. Position des ersten Sensors auf dem ersten Abschnitt. Die zweite Variable bestimmt die gemessene Lage bzw. Position des ersten Sensors in Bezug auf das Messsystem. Die dritte Variable bestimmt die Lage bzw. Position des zweiten Sensors in Bezug auf das Messsystem. Vorzugsweise werden die Variablen als homogene Transformationsmatrizen dargestellt und die erste Variable mit der zweiten Variablen multipliziert und das Ergebnis mit der Inversen der dritten Variablen multipliziert. Das Ergebnis dieser Gleichung bestimmt die Lage des zweiten Sensors auf dem zweiten Abschnitt in Bezug auf den ersten Abschnitt.

Die mathematische Gleichung kann in einer bevorzugten Ausführungsform beispielsweise wie folgt aussehen:

$$^{Tcp}T_N = {}^{Tcp}T_H * {}^{H}T_{Mess} * ({}^{N}T_{Mess})^{-1} - \text{für homogene Transformationsmatrizen}$$

Dabei gilt:

N        Nebensensor
H        Hauptsensor
TCP      Instrumentenspitze
Mess     Messwert
T        homogene Transformationsmatrize
$^{Tcp}T_N$     räumliche Beziehung zwischen der Instrumentenspitze (TCP) und dem Nebensensor
$^{Tcp}T_H$     räumliche Beziehung zwischen der Instrumentenspitze (TCP) und dem Hauptsensor
$^{H}T_{Mess}$     Lagemessdaten des Hauptsensors
$^{N}T_{Mess}$     Positionsmessdaten des Nebensensors

In einer bevorzugten Ausführungsform umfasst die Erfindung in einer vorteilhaften Weiterentwicklung das Hinterlegen von Daten, welche die Lage des zweiten Sensors in Bezug auf den ersten Abschnitt bei möglichen Anwendungen wiedergeben.

Diese hinterlegten Daten können beispielsweise als Funktion $T_{soll}$ bezeichnet werden und die Beziehungen zwischen Instrumentenspitze und Nebensensoren beschreiben.

Die vorliegende Erfindung betrifft insbesondere die Positionsbestimmung von Instrumenten, die sich während ihrer Anwendung verformen, sei dies aufgrund einer beabsichtigten oder einer nicht beabsichtigen Verformung oder Deformation des Instrumentes. Eine Verformung wird hier in dem Sinn beschrieben, dass der zweite Abschnitt seine Lage in Bezug auf den ersten Abschnitt verändert. Je nach Geometrie und Materialbeschaffenheit des Instrumentes, nach physikalischen Umgebungsparametern wie beispielsweise Temperatur und Druck sowie nach den voraussichtlich angreifenden Kräften und Momenten an dem Instrument kann es möglich sein, die voraussichtliche Verformung mathematisch zu beschreiben oder mit ausreichender Genauigkeit zu approximieren. Dies bedeutet, es können Positionsdaten hinterlegt werden, die beispielsweise die möglichen Raumkoordinaten oder relativen Koordinaten zwischen erstem und zweitem Abschnitt oder zwischen erstem oder zweitem Sensor oder zwischen einem der Abschnitte und einem der Sensoren bei einer gewollten oder ungewollten Verformung beschreiben. Mittels der hinterlegten Daten kann geprüft werden, ob gemessene Lageveränderungen oder Deformationen mit den hinterlegten Daten übereinstimmen oder sich in deren Rahmen bewegen. Bei Abweichungen kann die Höhe der Abweichung ermittelt werden, um anschließend verschiedene Ursachen zu prüfen oder bestimmte Ergebnisse der Positionsermittlung zu verwerfen.

Diese Daten können beispielsweise auf einer Recheneinheit bzw. einem Computer gespeichert sein oder werden. Dieser Computer kann bevorzugt zugleich zur Aufnahme der Messdaten der Sensoren und der nachfolgenden Bestimmung der Lage der Sensoren genutzt werden.

Weiterhin ist es möglich, mehrere Instrumente und mehrere hinterlegte Datensätze bei einer Anwendung zu nutzen. Die Zuordnung der Instrumente und Datensätze erfolgt dann beispielsweise mittels Identifikationsnummern der Instrumente und den dazugehörigen hinterlegten Daten. Die Zuordnung kann beispielweise mittels einer Recheneinheit und einer Datenbank durchgeführt werden. Die hinterlegten Daten können in einem Speicher in der Recheneinheit oder in einem externen Speicher hinterlegt werden. Das Verfahren zum Erzeugen der Positionsdaten kann dann auf die verschiedenen Instrumente und hinterlegten Daten angewendet werden.

Die hinterlegten Daten können jedoch auch aus einem dem Verfahren vorausgegangenen Verfahren stammen. Sie können z.B. vom Hersteller des verwendeten Instrumentes bereits bei dessen Erwerb mitgeliefert sein und in gespeicherter Form vorliegen.

In einer wiederum weiter bevorzugten Ausführungsform des Verfahrens dienen die hinterlegten Daten (unabhängig davon, ob sie mittels des erfindungsgemäßen Verfahrens oder losgelöst von diesem erhoben und hinterlegt wurden), welche die zulässigen Lagen des zweiten Sensors in Bezug auf den ersten Abschnitt bei möglichen Anwendungen des Instrumentes wiedergeben, zu einer Evaluierung der Daten, die mittels der mathematischen Formel aufgrund tatsächlicher Messungen erhoben wurden.

So können beispielsweise drei Parameter, welche die Messdaten des ersten und zweiten Sensors sowie der Position des zweiten Sensors in Bezug auf den ersten Abschnitt beschreiben, bestimmt werden. Im Idealfall sollten diese Daten gleich sein. Dies würde bedeuten, dass die hinterlegten Daten, also die theoretisch möglichen Positionsdaten des Instrumentes, mit den aufgrund von Messdaten ermittelten Positionsdaten exakt übereinstimmen.

Tatsächlich können jedoch diese theoretisch ermittelten Positionsdaten und die aufgrund von im Benutzungsfall tatsächlichen vorliegender Messdaten ermittelten Positionsdaten voneinander abweichen. Daher wird in einer erfindungsgemäßen Ausführungsform des Verfahrens vorgeschlagen, zunächst eine absolute Differenz zwischen diesen Daten zu bestimmen. Da es sich nur um ein Maß zur Bestimmung der absoluten Abweichung, nicht aber der Richtung der Abweichung (theoretische Daten gegenüber den Messdaten) handelt, wird vorzugsweise wiederum nur die absolute Differenz betrachtet. Entsprechend ausgeprägte Abweichungen können einen ersten Hinweis auf verschiedene Einflussgrößen, Messfehler, Störgrößen oder andere Artefakte bei der Positionsbestimmung mittels der Messdaten geben.

Daher wird in einer weiter bevorzugten Ausführungsform des Verfahrens weiterhin vorgeschlagen, einen oberen Grenzwert der absoluten Differenz nach jeder Messreihe bzw. dem Erzeugen von Positionsdaten eines Instrumentes festzulegen. Oberhalb dieses Grenzwertes können die aufgrund der Messungen erhobenen Positionsdaten als mit hoher Wahrscheinlichkeit durch Messfehler, Störgrößen oder andere Artefakte beeinflusste Daten interpretiert werden. Es ist in diesem Fall vorteilhaft möglich, geeignete Konsequenzen aus dieser Erkenntnis zu ziehen.

In einer wiederum weiter bevorzugten Ausführungsform des Verfahrens wird ein Instrument verwendet, bei welchem ein Gelenk- oder Biegeelement zwischen den beiden Abschnitten (bzw. zwischen zwei Abschnitten allgemein) vorgesehen ist. Ein Biegeelement kann beispielsweise ein Kugelgelenk sein. Dies hat den Vorteil, dass eine mathematische Beschreibung der möglichen Positionen des zweiten Abschnitts bezogen auf den ersten Abschnitt z.B. als elliptisches Paraboloid aufgrund der Kenntnis der - vor allem bei geeigneter Auswahl des Biegeelements - begrenzten Biegemöglichkeit des Biegeelements eindeutig und ggf. besonders einfach abgebildet werden kann. Diese mathematische Beschreibung kann wiederum in einem Rechner hinterlegt und mit ermittelten Messpositionen verglichen werden.

In einer wiederum weiter bevorzugten Ausführungsform des Verfahrens wird ein Instrument verwendet, bei welchem eine räumliche Begrenzung des zweiten Abschnitts vorgesehen ist. Diese räumliche Begrenzung kann beispielsweise konstruktiv erzielt werden, indem das Instrument einen mechanischen Anschlag aufweist. Dadurch werden die möglichen Positionen des Instrumentes begrenzt, was in der Anwendung beispielsweise bei der Führung durch enge Röhren vorteilhaft sein kann. Diese räumliche Begrenzung wird in der mathematischen Beschreibung der möglichen Positionen als Randbedingungen berücksichtigt.

In einer wiederum weiter bevorzugten Ausführungsform weist das verwendete Instrument eine fixe Verbindung zwischen dem ersten und zweiten Abschnitt auf. Dadurch werden die möglichen Positionen des zweiten Abschnitts deutlich eingeschränkt. Die möglichen Positionen werden dann beispielsweise durch äußere Kräfte und Momente auf das starre Instrument beeinflusst. Diese oft ungewollten Deformationen während des Einsatzes sind meist reversibel (elastisch), jedoch abhängig von den Materialeigenschaften, den physikalischen Umgebungsparametern wie Temperatur und Druck und den auftretenden Kräften und Momenten.

In einer wiederum weiter bevorzugten Ausführungsform werden die räumlichen Zuordnungen der Sensoren und möglicher Bezugspunkte sowohl bei den Messungen als auch bei den mathematischen Beschreibungen als homogene Transformationsmatrizen dargestellt. Damit können alle räumlichen Beziehungen sowohl für die Messpositionen als auch für die mathematischen Beschreibungen hinlänglich dargestellt bzw. mathematisch abgebildet werden.

In einer wiederum weiter bevorzugten Ausführungsform werden die mittels der Sensoren gemessenen Positionsdaten und einer möglichen nachfolgenden Weiterverarbeitung in einer Steuereinheit oder in einem Computer auf einer geeigneten Ausgabeeinrichtung ausgegeben. Eine derartige Ausgabeeinrichtung kann beispielsweise ein Monitor sein.

Hierbei kann es sinnvoll sein, Messdaten, die signifikant von zuvor theoretisch ermittelten möglichen Positionsdaten abweichen und außerhalb eines Grenzwertes liegen, der zuvor festgelegt worden ist, nicht auszugeben. Dies kann beispielsweise durch einen Schwellwertfilter in einer Steuereinheit realisiert werden. Dies hat den Vorteil, dass eine - insbesondere offensichtliche - Verfälschung der Positionsdaten durch Werte, die oberhalb eines festgelegten Grenzwertes liegen, auf einer Anzeigeeinheit nicht ausgegeben werden und damit nicht sichtbar ist.

Umfasst ist auch das Vorsehen von mehr als nur einem Schwellenwert.

Eine weitere Möglichkeit, die vorgeschlagen wird, besteht darin, Werte, die außerhalb eines festgelegten Grenzwertes liegen, mit einem geeigneten Verfahren zu korrigieren und anschließend auszugeben. Mögliche Korrekturalgorithmen sind dem Fachmann bekannt und werden hier nicht weiter beschrieben. Gleichwohl sind solche in Verbindung mit den hierin beschriebenen Merkmalen und Merkmalskombinationen ausdrücklich als offenbart zu verstehen.

Sowohl die Nichtausgabe von Messwerten oberhalb eines Grenzwertes als auch die Korrektur und anschließende Ausgabe der Messwerte zur Positionsbestimmung des Instrumentes können dazu geeignet sein, die Positionsbestimmung von Instrumenten in ihrer Genauigkeit zu verbessern und die Zuverlässigkeit bezüglich der Ausgabe der tatsächlichen Position des Instrumentes vorteilhaft zu erhöhen. Die - vor allem unbemerkte - Beeinflussung durch Gegenstände im Umfeld des Instrumentes, genauer im Messbereich der Sensoren auf dem Instrument, wird durch das beschriebene Verfahren vorteilhaft verringert. Bei den beschriebenen elektromagnetischen Sensoren (Spulenelementen) gilt dies beispielsweise für die Beeinflussung von Gegenständen, die ferromagnetische Eigenschaften aufweisen. Die Feldstörung durch diese Gegenstände oder ihr Einfluss auf eine Positionsermittlung wird durch das genannte Verfahren, besonders für nicht-starre Instrumente, die für gewollte Deformationen geeignet sind, und für starre, jedoch deformierbare Instrumente mit Lagesensoren verringert.

In einer wiederum weiter bevorzugten Ausführungsform wird das Verfahren bei medizinischen Instrumenten angewendet. Beispielsweise in der Mund-, Kiefer-, Gesichtschirurgie als auch im Hals-Nasen-Ohren Bereich kann es während operativen Eingriffen sehr wichtig sein, die genaue Position von Operationsinstrumenten zu kennen, um nicht Gefäße, Nerven oder andere sensible Bereiche zu verletzen. Gleichzeitig werden bei derartigen Operationen eine Vielzahl von Instrumenten, Überwachungs- und Diagnosegeräten und anderen Hilfsmitteln für die Operation eingesetzt. Oftmals besteht eine Vielzahl von diesen Instrumenten und Geräten aus metallischen Werkstoffen, beispielsweise um eine Sichtbarkeit auf Computertomographien zu erreichen. Ein weiterer Grund zum heute verbreiteten Einsatz von metallischen Werkstoffen bei Operationsinstrumenten ist die gute Sterilisierbarkeit, die gerade im medizinischen Bereich von großer Bedeutung ist. Andererseits finden neue operative Operationstechniken wie beispielsweise die minimal-invasive Chirurgie immer größere Anwendungsfelder, die oft ein genaues Navigieren und Führen von Instrumenten in nicht direkt einsehbaren Gebieten erfordern. Auch in den zuvor genannten Einsatzbereichen bietet das erfindungsgemäße Verfahren große Vorteile gegenüber dem aus dem Stand der Technik Bekannten.

Das verwendete Instrument kann beispielsweise eine Ultraschallsonde (Rektal-, Vaginalsonde, intravaskuläre Sonde), ein Katheter, ein Sauger oder ein Zeigeinstrument sein. Oft kann es vorteilhaft sein, wenn zumindest ein Abschnitt ein geometrisch definiertes Ende aufweist, um beispielsweise als Zeigeinstrument geeignet anwendbar zu sein. Bei einer Ultraschallsonde kann es vorteilhaft sein, wenn die Messsonde am Ende eines Abschnittes angeordnet ist. Je nach Sondenform kann das Ende rund, eckig oder spitz ausgeführt werden. Instrumente mit mehr als zwei Abschnitten sind ebenfalls möglich, wenn beispielsweise mehrere Funktionen, Geometrien oder Messaufgaben durchgeführt werden sollen.

Mit Hilfe des Verfahrens ist es vorteilhaft möglich, die Positionsbestimmung von Instrumenten, die sich während der Anwendung verformen, zu beschreiben, und/oder erkannte Artefakte mitzuteilen oder in einer Positionsbestimmung zu berücksichtigen.

Vorteilhafter Weise können in bestimmten Ausführungsformen Störungen auf die Lagemessung, seien es Verformungen des Instruments oder Störungen durch Störobjekte, die das Messverfahren beeinflussen, erkannt und eliminiert werden.

Für Instrumente, die sich während der Anwendung verformen, sei es eine beabsichtigte oder eine nicht beabsichtige Verformung oder Deformation des Instrumentes, ist bisher kein Verfahren bekannt, um Störungen auf die Lagemessung hervorgerufen durch Störobjekte getrennt von den Deformationen bzw. Biegung zu erkennen und zu eliminieren. Das hierfür bestehende Bedürfnis wird von manchen erfindungsgemäßen Ausführungsformen vorteilhaft befriedigt.

Insbesondere ist es mittels des Verfahrens vorteilhaft möglich, auf das Vorliegen - insbesondere für den Anwender oder Operateur - von nicht sichtbaren Gegenständen im räumlichen Messdatenbereich aufmerksam zu machen, welche die messtechnische Bestimmung der Lage der Sensoren beeinflussen. Beruhen die Sensoren beispielsweise auf elektromagnetischen Wirkprinzipien, so kann eine derartige Beeinflussung durch Gegenstände aus ferromagnetischen Materialien hervorgerufen werden. Aber auch andere metallische Werkstoffe können das Messergebnis beeinflussen. Die Höhe der Beeinflussung, d. h. die quantitative Veränderung der Messsignale, hängt von verschiedenen Faktoren ab, wie beispielweise der unmittelbaren Nähe der Gegenstände zu den Sensoren. Die messtechnische Positionsbestimmung der Sensoren und damit des gesamten Instrumentes kann sich demnach mit dem Vorhandensein derartiger Gegenstände verändern. Diese Phänomene können zu Artefakten bei der Bestimmung der Position des Instrumentes oder eines Abschnitts hiervon (z.B. seiner Spitze) führen. Mittels des Verfahrens können derartige Störungen jedoch vorteilhafter Weise erkannt und/oder korrigiert werden.

Die Aufgabe wird auch gelöst durch die Vorrichtung gemäß Anspruch 7. Die Aufgabe wird ferner gelöst durch ein Computerprogramm-Produkt mit Programmcode gemäß Anspruch 13, ein Computerprogramm gemäß dem Anspruch 14 und ein digitales Speichermedium gemäß Anspruch 15 . Da die hiermit jeweils erzielbaren Vorteile ungeschmälert jenen oben erläuterten entsprechen, wird zur Vermeidung von Wiederholung an dieser Stelle ausdrücklich auf ihre obige Diskussion verwiesen. Dasselbe gilt für die oben dargelegte Beschreibung des Instrumentes oder andere strukturelle Ausgestaltungen und Merkmale der Vorrichtung.

Dabei kann die Vorrichtung eine Instrumentenspitze als ein Ende des ersten Abschnitts als Bezugspunkt zum Erzeugen der Positionsdaten des Instrumentes aufweisen. Dies hat den Vorteil, dass beispielsweise bei der Verwendung als Zeigeinstrument die Instrumentenspitze als Bezugspunkt besonders geeignet ist.

Weiterhin kann die Vorrichtung einen ersten und einen zweiten Abschnitt aufweisen, der jeweils starr ausgebildet ist. Dies hat den Vorteil, dass bei einer Deformation jeweils nur ein Sensor auf jedem Abschnitt für die Positionsbestimmung des Instrumentes ausreichend ist.

Im Folgenden wird das Verfahren anhand der beigefügten Zeichnung, in welcher identische Bezugszeichen gleiche oder ähnliche Bauteile bezeichnen, exemplarisch erläutert. In den zum Teil stark vereinfachten Figuren gilt:

Fig. 1 zeigt eine schematische Übersicht einer beispielhaften Anordnung zur Durchführung des Verfahrens;
Fig. 2 zeigt eine schematische Übersicht einer weiteren beispielhaften Anordnung zur Durchführung des Verfahrens; und
Fig. 3 zeigt eine schematische Übersicht einer weiteren beispielhaften Anordnung zur Durchführung des Verfahrens.

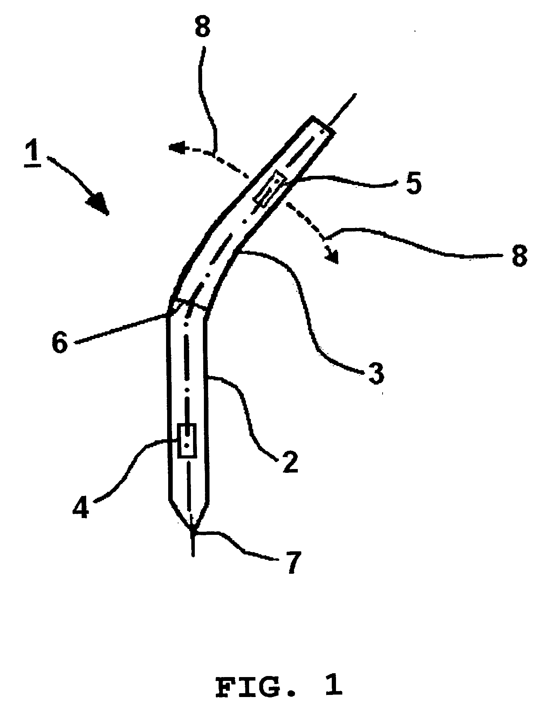

Fig. 1 zeigt ein zylinderförmiges Instrument 1 mit einem ersten Abschnitt 2 und einem zweiten Abschnitt 3. Am ersten Abschnitt 2 befindet sich ein erster Sensor 4, am zweiten Abschnitt 3 befindet ein zweiter Sensor 5. Beide Sensoren 4, 5 werden schematisch auf der Oberfläche des Instrumentes gezeigt, können jedoch auch in einem Inneren (nicht dargestellt) des jeweiligen Abschnitts 1, 2 liegen.

Der erste Abschnitt 2 ist fest mit dem zweiten Abschnitt 3 verbunden, beispielsweise durch eine Klebenaht 6. Es ist jedoch auch möglich, dass die beiden Abschnitte 2 und 3 aus einem Teil bestehen, dann wäre keine Trennfuge sichtbar. Ein Ende des ersten Anschnitts 2 wird als kegelförmige Instrumentenspitze 7 dargestellt.

Das Instrument 1 wird in Fig. 1 in einem deformierten Zustand gezeigt. Der Abschnitt 3 ist gegenüber dem Abschnitt 2 deutlich gebogen dargestellt. Die Biegestelle, um die der Abschnitt 3 gegenüber Abschnitt 2 gebogen ist, ist die Klebenaht 6. Sowohl der Abschnitt 2 als auch der Abschnitt 3 sind dagegen weitgehend starr ausgebildet. Die Sensoren 4 und 5 befinden sich in den Bereichen der Abschnitte 2 und 3, die nicht in sich gebogen sind. Der Übergang zwischen diesem starren, nicht in sich deformierten Bereich der Abschnitte 2 und 3 und dem in sich gebogenen Bereich im Bereich der Klebenahtstelle 6 ist fließend und wird nicht klar abgegrenzt.

Mögliche Deformationszustände des Instrumentes 1 werden anhand der möglichen räumlichen Positionen des Sensors 5 dargestellt und werden als Biegelinie 8 bezeichnet. Dabei können die möglichen Positionen des Sensors 5 anhand geeigneter mathematischer Funktionen beschrieben werden, für die hier als Bezugspunkt die Instrumentenspitze 7 gewählt wird.

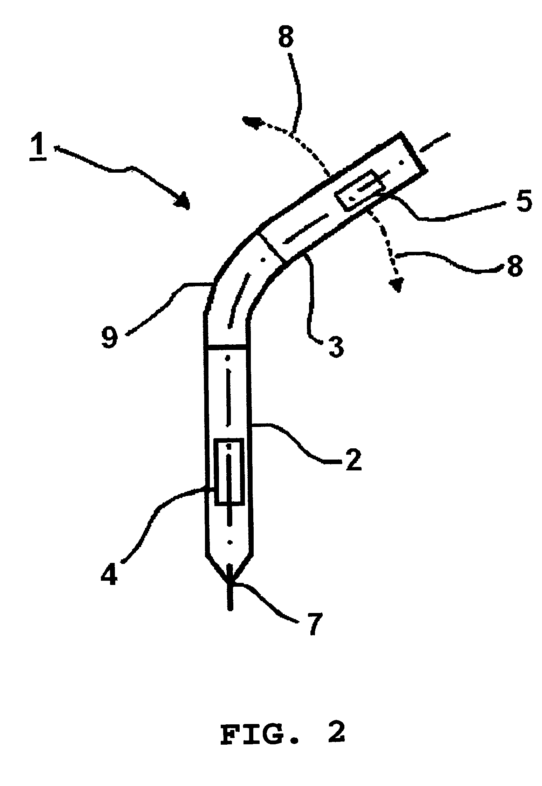

In Fig. 2 wird ein ebenfalls zylinderförmiges Instrument 1 mit einem ersten Abschnitt 2 und einem zweiten Abschnitt 3 dargestellt. Die Sensoren 4 und 5 befinden sich in der gleichen Art und Weise wie in Fig. 1 auf den Abschnitten 2 und 3. Im Unterschied zu Fig. 1 wird hier jedoch ein Biegeelement 9 zwischen den Abschnitten 2 und 3 angeordnet. Dieses Biegeelement 9 hat die Funktion, die Deformation bzw. die Biegung des Abschnittes 3 gegenüber der Instrumentenspitze 7 als Bezugspunkt des Abschnittes 2 in einer vorhersagbaren und mathematisch beschreibbaren Funktion wiedergeben zu können. Diese Funktion wird als mathematische Formel der Biegelinie 8 beschrieben.

Als Biegeelemente 9 können beispielsweise mechanische Gelenke verwendet werden. Bei der Verwendung eines Kugelgelenkes kann die Biegelinie 9 als elliptisches Paraboloid mathematisch beschrieben werden. Weiterhin möglich sind beispielsweise auch elastische Polymermaterialien als Biegeelemente 9, die gezielt Verformungen in diesem Bereich zulassen würden.

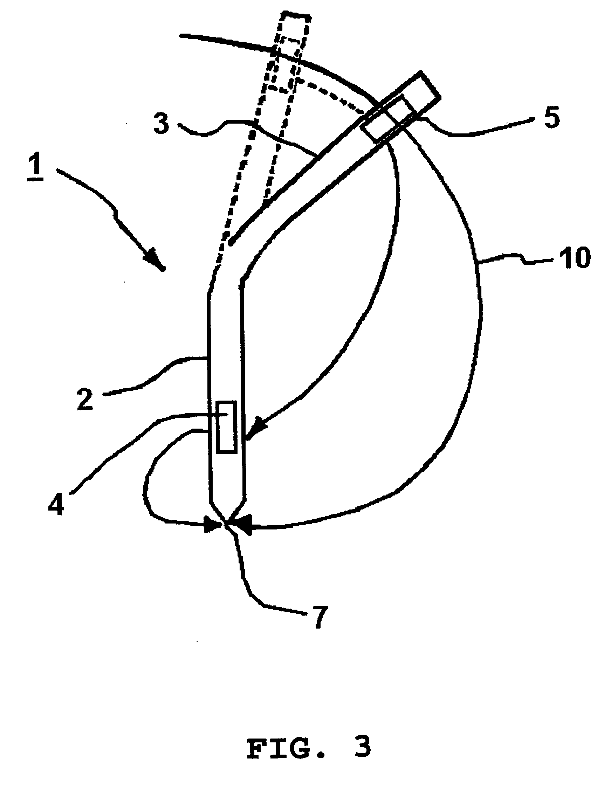

In Fig. 3 wird eine Weglinie 10 dargestellt, welche die möglichen Positionen des Nebensensors aufzeigen.

## Patentansprüche

1. Verfahren zum Erzeugen von Positionsdaten und zum Erkennen von Deformationen eines medizinischen Instrumentes (1) mit wenigstens einem ersten Abschnitt (2) mit wenigstens einem ersten Sensor (4) und wenigstens einem zweiten Abschnitt (3) mit wenigstens einem zweiten Sensor (5); wobei das Verfahren ein messtechnisches Bestimmen der Lage des ersten und zweiten Sensors (4, 5) umfasst;
mit den Schritten

- Hinterlegen von Daten, welche zulässige Lagen des zweiten Sensors (5) in Bezug auf den ersten Abschnitt (2) wiedergeben;
- rechnerisches Bestimmen der Lage des zweiten Sensors (5) in Bezug auf den ersten Abschnitt (2);
- Vergleichen der rechnerisch bestimmten Lage des zweiten Sensors (5) in Bezug auf den ersten Abschnitt (2) mit den hinterlegten Daten;
- Bestimmen der absoluten Differenz zwischen der rechnerisch bestimmten Lage des zweiten Sensors (5) in Bezug auf den ersten Abschnitt (2) und den hinterlegten Daten; und
- Festlegen eines oberen Grenzwertes für die absolute Differenz; wobei hierfür

das rechnerische Bestimmen der Lage des zweiten Sensors (5) in Bezug auf den ersten Abschnitt (2) auf mindestens zwei Arten erfolgt, wobei auf eine der beiden Arten nicht die messtechnisch bestimmten Lagen des ersten und zweiten Sensors (4, 5) verwendet werden, die während der Anwendung erfasst werden.

2. Verfahren nach einem der vorangegangenen Ansprüche, wobei die Instrumentenspitze (7) der Bezugspunkt zum Erzeugen der Positionsdaten des ersten Abschnitts (2) des Instrumentes (1) ist.

3. Verfahren nach einem der vorangegangenen Ansprüche, wobei die räumliche Zuordnung wenigstens des ersten und/oder zweiten Abschnitts (2, 3) und wenigstens des ersten und/oder zweiten Sensors (4, 5) als homogene Transformationsmatrizen beschrieben werden.

4. Verfahren nach einem der vorangegangenen Ansprüche, wobei die Positionsdaten des Instrumentes (1) auf einer Ausgabeeinrichtung ausgegeben werden.

5. Verfahren nach einem der vorangegangenen Ansprüche, wobei die Positionsdaten außerhalb der Grenzwerte als Störsignale erkannt und nicht ausgegeben werden.

6. Verfahren nach einem der vorangegangenen Ansprüche, wobei die Positionsdaten außerhalb der Grenzwerte als Störsignale erkannt, zunächst korrigiert und anschließend ausgegeben werden.

7. Vorrichtung zum Durchführen des Verfahrens nach wenigstens einem der Ansprüche 1 bis 6, welche ein medizinisches Instrument aufweist, mit einer Recheneinheit zur Verarbeitung der Messsignale und als Schnittstelle zu einer Ausgabeeinheit und einer Speichereinrichtung zum Hinterlegen der Daten und mit einem Lagemesssystem, konfiguriert zum Durchführen des Verfahrens nach einem der Ansprüche 1 bis 6.

8. Vorrichtung nach Anspruch 7, wobei der erste und zweite Abschnitt (2, 3) des Instrumentes (1) jeweils starr ausgebildet sind.

9. Vorrichtung nach einem der Ansprüche 7 bis 8, wobei der erste und zweite Abschnitt (2, 3) des Instrumentes (1) flexibel ausgebildet sind.

10. Vorrichtung nach einem der Ansprüche 7 bis 9, wobei der erste und zweite Abschnitt (2, 3) des Instrumentes (1) durch ein Biegeelement verbunden sind.

11. Vorrichtung nach einem der Ansprüche 7 bis 10, wobei der zweite Abschnitt (3) des Instrumentes (1) räumlich begrenzt ausgebildet wird.

12. Vorrichtung nach einem der Ansprüche 7 bis 13, wobei der erste und zweite Abschnitt (2, 3) des Instrumentes (1) fest verbunden sind.

13. Computerprogramm-Produkt mit Programmcode, der dazu konfiguriert ist, das Verfahren nach einem der Ansprüche 1 bis 6 auszuführen, wenn der Programmcode auf einem Computer ausgeführt wird.

14. Computerprogramm, das Anweisungen umfasst, die dazu konfiguriert sind, das Verfahren nach einem der Ansprüche 1 bis 6 auszuführen, wenn das Computerprogramm auf einem Computer ausgeführt wird.

15. Digitales Speichermedium, insbesondere Diskette, CD oder DVD, mit elektrisch auslesbaren Steuersignalen, die derart mit einem programmierbaren Computersystem zusammenwirken können, dass die Schritte eines Verfahrens gemäß einem der Ansprüche 1 bis 6 durch das Computersystem ausgeführt werden.

## Claims

1. A method for generating position data and detecting deformations of a medical instrument (1) with at least one first section (2) with at least one first sensor (4) and at least one second section (3) with at least one second sensor (5), whereby the method encompasses determining the position of the first and second sensor (4, 5) metrologically; with the steps

   - storing data which reflect allowable positions of the second sensor (5) with regard to the first section (2);
   - mathematically determining the position of the second sensor (5) with regard to the first section (2);

- comparing the mathematically determined position of the second sensor (5) with regard to the first section (2) with the data on file;
- determining the absolute difference between the mathematically determined position of the second sensor (5) with regard to the first section (2) and the data on file; and
- setting up an upper limit value for the absolute difference;

wherein for this purpose the mathematical determination of the position of the second sensor (5) with regard to the first section (2) takes place in at least two ways, wherein with one of the two ways the metrologically determined position of the first and second sensors (4, 5) determined during use are not used.

2. The method according to one of the preceding claims, wherein the tip of the instrument (7) is the reference point for generating the position data of the first section (2) of the instrument (1).

3. The method according to one of the preceding claims, wherein the spatial allocation of at least the first and/or the second section (2, 3) and at least the first and/or the second sensor (4, 5) are described as homogenous transformation matrices.

4. The method according to one of the preceding claims, wherein the position data of the instrument (1) are output to an output device.

5. The method according to one of the preceding claims, wherein the position data outside the limit values are detected to be interfering signals and are not output.

6. The method according to one of the preceding claims, wherein the position data outside the limit values are detected to be interfering signals, are corrected first and subsequently output.

7. An apparatus configured for executing the method according to at least one of the claims 1 to 6, comprising a medical instrument, with a processing unit for processing the measurement signals and as an interface to an output unit, with a storage device for filing the data, and with a position measuring system, configured for executing the method according to one of the claims 1 to 6.

8. The apparatus according to claim 7, wherein the first and the second section (2, 3) of the instrument (1) are each rigid.

9. The apparatus according to one of the claims 7 to 8, wherein the first and second section (2, 3) of the instrument (1) are flexible.

10. The apparatus according to one of the claims 7 to 9, wherein the first and the second section (2, 3) of the instrument (1) are connected by means of a bending element.

11. The apparatus according to one of the claims 7 to 10, wherein the second section (3) of the instrument (1) is spatially limited.

12. The apparatus according to one of the claims 7 to 13, wherein the first and the second section (2, 3) of the instrument (1) are firmly connected.

13. A computer program product with a program code which is configured for executing the method according to one of the claims 1 to 6 when the program code is executed on a computer.

14. A computer program which encompasses instructions which are configured for executing the method according to one of the claims 1 to 6 when the computer program is executed on a computer.

15. A digital storage medium, in particular a disk, CD, or DVD, with electrically readable executing signals which are configured so as to cooperate with a programmable computer system for executing the steps of a method according to one of the claims 1 to 6 by means of the computer system.

**Revendications**

1. Un procédé pour générer des données de position et pour détecter des déformations d'un instrument médical (1) ayant au moins une première partie (2) avec au moins un premier capteur (4) et au moins une seconde partie (3) avec au moins un second capteur (5), où le procédé comprend une détermination par technique de mesure de la position du premier et du second capteur (4, 5).
comprenant les étapes

   - enregistrer des données représentant les positions admissibles du second capteur (5) par rapport à la première partie (2) ;
   - déterminer mathématiquement la position du second capteur (5) par rapport à la première partie (2) ;
   - comparer la position du second capteur (5) relative à la première partie (2) qui a été déterminée mathématiquement avec les données enregistrées ;
   - déterminer la différence absolue entre la position du second capteur (5) relative à la première partie (2) qui a été déterminée mathématiquement et les données enregistrées ; et
   - définir une valeur limite supérieure pour la différence absolue ;

   où à cet effet la détermination mathématique de la position du second capteur (5) par rapport à la première partie (2) a lieu au moins de deux façons, et où pour l'une des deux façons, les positions du premier et du deuxième capteur (4, 5) déterminées par technique de mesure qui ont été saisies pendant l'usage ne sont pas utilisées.

2. Le procédé selon l'une des revendications précédentes, où la pointe de l'instrument (7) est le point de référence pour générer les données de position de la première partie (2) de l'instrument (1).

3. Le procédé selon l'une des revendications précédentes, où l'affectation spatiale d'au moins la première et/ou seconde partie (2, 3) et d'au moins le premier et/ou second capteur (4, 5) sont décrites par des matrices de transformation homogènes.

4. Le procédé selon l'une des revendications précédentes, où les données de position de l'instrument (1) sont transmises à un dispositif de sortie.

5. Le procédé selon l'une des revendications précédentes, où les données de position hors des valeurs limites sont détectées comme étant des signaux perturbateurs et ne sont pas transmises.

6. Le procédé selon l'une des revendications précédentes, où les données de position hors des valeurs limites sont détectées comme étant des signaux perturbateurs, sont tout d'abord corrigées puis postérieurement transmises.

7. Un appareil configuré pour exécuter le procédé selon au moins l'une des revendications 1 à 6, comprenant un instrument médical, avec une unité de traitement pour traiter les signaux de mesure et pour agir comme interface pour une unité de sortie, avec un dispositif de stockage pour enregistrer les données et avec un système de mesure de position, configuré pour exécuter le procédé selon l'une des revendications 1 à 6.

8. Un appareil selon la revendication 7, où la première et la seconde partie (2, 3) de l'instrument (1) sont chacune formées de manière rigide.

9. Un appareil selon l'une des revendications 7 à 8, où la première et la seconde partie (2,3) de l'instrument (1) sont chacune formées de manière flexible.

10. Un appareil selon l'une des revendications 7 à 9, où la première et la seconde partie (2,3) de l'instrument (1) sont connectées au moyen d'un élément de flexion.

11. Un appareil selon l'une des revendications 7 à 10, où la seconde partie (3) de l'instrument (1) est conçue de manière à être spatialement limitée.

12. Un appareil selon l'une des revendications 7 à 13, où la première et la seconde partie (2, 3) de l'instrument (1) sont solidement connectées.

13. Un produit logiciel avec un code de programme configuré pour exécuter le procédé selon l'une des revendications 1 à 6, lors de l'exécution du code de programme sur un ordinateur.

14. Un programme informatique comprenant des instructions configurées pour exécuter le procédé selon l'une des revendications 1 à 6, lors de l'exécution du programme informatique sur un ordinateur.

15. Un support de stockage numérique, en particulier un disque, CD ou DVD, avec des signaux d'exécution lisibles électriquement et pouvant interagir avec un système informatique programmable pour exécuter le procédé selon l'une des revendications 1 à 6 au moyen du système informatique.

FIG. 1

FIG. 2

FIG. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2004017834 A1
- DE 102006052886 A1
- US 20080294034 A1
- US 20050228270 A1